(19) Europäisches Patentamt
European Patent Office
Office européen des brevets

(11) Veröffentlichungsnummer : **0 019 772**
**B1**

(12) # EUROPÄISCHE PATENTSCHRIFT

(45) Veröffentlichungstag der Patentschrift : **15.12.82**

(51) Int. Cl.³ : **C 07 C 47/127**, C 07 C 45/38

(21) Anmeldenummer : **80102526.3**

(22) Anmeldetag : **08.05.80**

(54) **Verfahren zur kontinuierlichen Herstellung von Glyoxal.**

(30) Priorität : **02.06.79 DE 2922599**

(43) Veröffentlichungstag der Anmeldung :
**10.12.80 (Patentblatt 80/25)**

(45) Bekanntmachung des Hinweises auf die Patenterteilung : **15.12.82 Patentblatt 82/50**

(84) Benannte Vertragsstaaten :
**BE CH DE FR GB LI NL**

(56) Entgegenhaltungen :
**EP A 0 003 343**

**CHEMICAL ABSTRACTS, Vol. 62, Nr. 1, 04-01-1965 M.S. BRODSKII et al., Columbus, Ohio, USA. Spalte 447c**
**CHEMICAL ABSTRACTS, Vol. 61, 20-07-1964 N.V. KUZNETSOV et al., Columbus, Ohio, USA**

(73) Patentinhaber : **BASF Aktiengesellschaft**
**Carl-Bosch-Strasse 38**
**D-6700 Ludwigshafen (DE)**

(72) Erfinder : **Sauer, Wolfgang, Dr.**
**Augusta-Anlage 42**
**D-6800 Mannheim (DE)**
Erfinder : **Hoffmann, Wolfgang, Dr.**
**Albert-Haueisen-Strasse 4**
**D-6710 Frankenthal (DE)**

**EP 0 019 772 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Verfahren zur kontinuierlichen Herstellung von Glyoxal

Die Erfindung betrifft ein Verfahren zur Herstellung von Glyoxal durch Oxidation von Glykol in Gegenwart eines Silberkatalysators und anschließender oder teilweiser Kondensation von Glykol, Glyoxal, Glykolaldehyd, Formaldehyd und/oder Wasser durch Behandlung mit Wasser oder wäßriger Glyoxallösung in feiner Zerteilung unter bestimmten Bedingungen der Temperatur und der Zerteilung der Behandlungsflüssigkeit.

Es ist aus Ullmanns Encyklopädie der technischen Chemie, Band 8, Seiten 250 bis 252, bekannt, daß eines der wichtigsten Verfahren zur Herstellung von Glyoxal die Oxidation von Äthylenglykol mit Luft ist. Als Bedingungen werden Temperaturen von 300 bis 325 °C Kupferoxid als Katalysator und der Zusatz von Halogenverbindungen genannt. Man erhält aus dem Reaktionsgemisch durch Absorption in einer Glyoxallösung oder in Wasser eine 32-prozentige Glyoxallösung. Ein solches Verfahren liefert Ausbeuten von höchstens 65 Prozent und Raum-Zeit-Ausbeuten von nur 0,04 bis 1,5 Gramm Glyoxal/$cm^3$ Katalysatorvolumen und Stunde. Eine Kondensation von Anteilen an Endstoff durch Versprühen von Wasser in den heißen Reaktionsgasen wird nicht beschrieben. Da in der Glyoxallösung zahlreiche Nebenprodukte, in der Hauptsache von stark saurem Charakter (loc. cit., Seite 251) enthalten sind, muß aus der Lösung mit Hilfe mehrerer fraktionierter und azeotroper Destillationen das Glyoxal abgetrennt werden.

Es ist aus Proceed. Acad. Sci. USSR, Che. Ser. (1964), Seiten 641 bis 643 bekannt, Silber als Katalysatoren auf Bimsstein und Aluminium als Träger zu verwenden. Das beste Ergebnis wurde mit Silberspiralen, einer Temperatur von 510 °C, einem Druck von 544 bis 816 mbar und einer Ausbeute von 69 Prozent erhalten. Der Endstoff fällt in Gestalt einer 25-gewichtsprozentigen, wäßrigen Lösung mit zusätzlich 5 bis 10 Prozent Glykol an ; über die Aufarbeitung der heißen Reaktionsgase werden keine näheren Angaben gemacht.

Die russische Patentschrift 136 352 beschreibt die Oxidation von Glykol bei 500 bis 700 °C, wobei als Katalysator Silber auf Aluminiumoxid (40 % Ag) verwendet wird. Die Ausbeute beträgt 61 Prozent, die Raum-Zeit-Ausbeute 12,8 Gramm Glyoxal pro Stunde und Gramm Katalysator. Das Verfahren hat den Nachteil, daß der Katalysator umständlich herzustellen ist und eine ziemlich verdünnte Glykollösung eingesetzt werden muß. Die Abtrennung des Endstoffes aus den Reaktionsgasen wird nicht näher beschrieben.

In der deutschen Auslegeschrift 10 32 732 wird dargelegt, daß man bei Verwendung von Kupfer und Silber als Katalysator einen Promotor, z.B. $TiO_2$ und $Mo_2O_5$, benötigt und zur Erhöhung der Ausbeute Hemmstoffe, z.B. HCl, $Cl_2$ oder Äthylendichlorid zusetzen muß. Als bestes Ergebnis erhält man danach eine Raum-Zeit-Ausbeute von 0,043 Gramm pro $cm^3$ und Stunde. Man kann nach der Lehre der Auslegeschrift das Ergebnis verbessern, wenn man das Silber auf Bimsstein, Silicagel oder Aluminiumoxid als Träger aufbringt. Das Verfahren wird zwischen 300 und 450 °C unter Verwendung eines Luft-Stickstoffgemisches mit einem Sauerstoffgehalt zwischen 1,6 und 5 Prozent durchgeführt. Bei einer Ausbeute von 55 Prozent wird eine Raum-Zeit-Ausbeute von 0,104 Gramm Glyoxal pro $cm^3$ Katalysatorraum und Stunde erreicht. Lediglich Beispiel 1 zeigt die Aufarbeitung der heißen Reaktionsgase. Man führt das Ausgangsgemisch im Reaktor von unten nach oben durch den Katalysator und anschließend die Reaktionsgase in zwei hintereinandergeschaltete, mit Raschigringen gefüllte Waschtürme, in denen Wasser umgepumpt wird.

Die deutsche Offenlegungsschrift 19 23 048 beschreibt die Herstellung von Glyoxal und verwendet als Katalysator 2 Komponenten (a und b) und zwar

a) Kupfer oder Silber und/oder Gold und außerdem
b) Germanium, Zinn, Blei, Stickstoff, Phosphor, Arsen, Antimon und/oder Wismut.

Bevorzugt ist Silber in Verbindung mit Zinn, Phosphor und/oder Arsen und insgesamt insbesondere Kupfer gegen- über Silber. Eine Reaktionstemperatur von ca. 177 bis 607 °C vorzugsweise von ca. 297 bis 447 °C wird angegeben. Das vergleichsweise zu zahlreichen Beispielen mit Kupferkatalysatoren einzige Beispiel mit einem Kupfer-freien Silberkatalysator (Silber/Phosphor) wird bei einer Temperatur von ca. 427 bis 447 °C durchgeführt. Aus den Angaben errechnet sich eine unbefriedigende Raum-Zeit-Ausbeute. Nachteilig ist ferner die umständliche Katalysatorherstellung. Die Reaktionsgase werden in einer Ausführungsform mittels einer Kühlstrecke (Beispiel 6) auf eine Temperatur unterhalb des Kondensationspunktes von Äthylenglykol und oberhalb des Kondensationspunktes von Glyoxal abgekühlt und so Athylenglykol von Glyoxal abgetrennt. 2,3-Dihydroxydioxan wird als Nebenprodukt gebildet. Verschiedene Reinigungsschritte schließen sich an. Auch bei weiteren Ausführungsformen wird offensichtlich eine Abkühlung mittels einer Kühlzone beschrieben. Ebenfalls zeigt Beispiel 1, daß der Endstoff aus den heißen Reaktionsgasen durch Abkühlung in zwei Kühlstrecken und nicht durch Vermischung mit Wasser kondensiert wird. Im Beispiel 5 werden die heißen Gase in eine Abschrecklösung in Gestalt einer rohen Glyoxallösung eingeleitet. Diese Lösung wird dann durch eine Abstreifsäule für Formaldehyd (105 °C), eine Ionenaustauschersäule und einen Dünnschichtverdampfer geführt. Lediglich zur Erhitzung und Spaltung des Nebenproduktes 2,3-Dihydroxydioxan (Beispiel 6), läßt man eine Lösung dieses Stoffes in Äthylenglykol in Form von feinen Tröpfchen auf einen Gasstrom, z.B. von Stickstoff, hoher Temperatur und Strömungsgeschwindigkeit aufprallen. Die Abtrennung des Formaldehyd kann durch Ausspülen der

wäßrigen Glyoxallösung mit einem Gemisch von Wasserdampf und Stickstoff bei erhöhter Temperatur erfolgen. Organische Säuren und gefärbte Verunreinigungen in den Reaktionsgasen müssen nach der Formaldehydabtrennung aus der wäßrigen Glyoxallösung mittels schwach basischer Anionenaustauscher abgetrennt werden, wobei sehr verdünnte Glyoxallösungen bevorzugt verwendet werden. Es wird angegeben, daß dank der vorgenannten Arbeitsweise größere Anteile an Verunreinigungen im Glyoxal vermieden werden.

In der deutschen Offenlegungsschrift 26 34 439 wird ein Katalysator verwendet, der aus Phosphor, kombiniert mit Cu und/oder Ag, besteht. Der Reaktion wird eine Bromverbindung zugesetzt, die dazu ausreicht, die Glyoxalausbeute zu erhöhen, die jedoch nicht so groß ist, daß die Glykolaldehydbildung merklich gesteigert wird oder die Umwandlung des Äthylenglykols auf weniger als etwa 90 Prozent absinkt. Inertgas wird zugesetzt. Die Raum-Zeit-Ausbeute beträgt nur 1,5 Gramm Glyoxal pro $cm^3$ Katalysator und Stunde. Ein weirerer Nachteil ist, daß der Katalysator nur umständlich regeneriert werden kann. Bezüglich der Aufarbeitung der Reaktionsgase wird lediglich angegeben, daß die Gase durch einen Wäscher geführt werden, wobei kondensierte Anteile in Form einer wäßrigen Lösung aufgefangen werden.

Es ist aus Ind. and Eng. Chem., Band 43 (1951), Seiten 786 bis 794, bekannt, daß man die Oxidationsgase nach der Oxidation über eine Rohrleitung zum Boden eines Waschturms (1. Wäscher), dann von unten nach oben durch den Waschturm und über eine Rohrleitung in eine Waschkolonne (2. Wäscher) führt. In den 1. Wäscher wird kalte Glyoxallösung über 3 Düsen senkrecht von oben im Gegenstrom zu den Gasen eingegeben. Der 2. Wäscher enthält eine Füllung von Raschigringen, durch die Glyoxallösung im Gegenstrom zum Abgas des 1. Wäschers fließt. Es wird darauf hingewiesen, daß erst der 2. Wäscher eine befriedigende Kondensation des Glyoxals aus den Oxidationsgasen erlaubt. Zur Abtrennung von Formaldehyd ist jedoch eine weitere Formaldehydabstreifkolonne notwendig. Beim Verlassen des Reaktionsraumes haben die Oxidationsgase eine Temperatur von 305 bis 325 °C. Wie eigene unveröffentlichte Untersuchungen ergeben, liegt die Temperatur der Oxidationsgase beim Eintritt in den Boden des 1. Wäschers bei 300 bis 320 °C. Die erhaltenen 32-gewichtsprozentigen Glyoxallösungen enthalten 12,8 Prozent unumgesetztes Glykol, 5,9 Prozent Formaldehyd, 1,6 Prozent Glykolsäure und 0,32 Prozent Ameisensäure.

Alle diese Verfahren sind mit Bezug auf einfachen und wirtschaftlichen Betrieb zusammen mit guter Ausbeute und Reinheit des Endstoffs sowie Abtrennung der Nebenstoffe unbefriedigend. Auch ist es nachteilig, daß häufig die Abkühlungsanlagen innerhalb kürzester Zeit verstopfen, so daß ein kontinuierlicher Betrieb erheblich beeinträchtigt wird. Die sich zum Teil in den anfallenden Reaktionsprodukten lösenden Crackprodukte und Polymeren verfärben die Glyoxallösungen derartig, daß sie den Ansprüchen der Weiterverarbeiter, insbesondere der Leder-, Papier- und Textilindustrie, nicht genügen.

Es wurde nun gefunden, daß man Glyoxal durch Oxidation von Glykol in Gegenwart eines Silberkatalysators bei erhöhter Temperatur, Abkühlung der heißen Reaktionsgase und Abtrennung des Endstoffs vorteilhaft herstellt, wenn man aus dem 450 bis 800 °C heißen Strom des dampfförmigen Reaktionsgemisches nach der Oxidation und höchstens eine Sekunde nach Austritt aus dem Katalysator mittels Wasser oder wäßriger Glyoxallösung von einer Temperatur von 0 bis 130 °C in Form von Tröpfchen von einem durchschnittlichen Durchmesser von 1 bis 2 000 Mikrometern Glyoxal, Glykol und/oder Glykolaldehyd, zusammen mit Wasser und gewünschtenfalls Formaldehyd, ganz oder teilweise kondensiert, wobei die Hauptmenge der Tröpfchen auf den Strom der Reaktionsgase mit einem Winkel von 2 bis 85° zur Stromachse auftrifft.

Die Umsetzung kann durch die folgenden Formeln wiedergegeben werden :

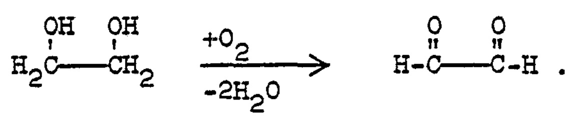


Im Vergleich zu den bekannten Verfahren liefert das Verfahren nach der Erfindung überraschend auf einfacherem und wirtschaftlicherem Wege ein besseres Gesamtergebnis mit Bezug auf Ausbeute, Raum-Zeit-Ausbeute und Reinheit des Endstoffs sowie Abtrennung der Nebenstoffe. Die Raum-Zeit-Ausbeute ist vergleichsweise besser. Je nach Wahl der erfindungsgemäßen Bedingungen erhält man wäßrige Glyoxallösungen von bis zu 65 Gewichtsprozent, vorzugsweise von 30 bis 50 Gewichtsprozent, insbesondere 38 bis 42 Gewichtsprozent. Weitere Vorteile des erfindungsgemäßen Verfahrens sind die gleichzeitige leichtere Entfernung der bei der Reaktion entstandenen Nebenstoffe, insbesondere des Formaldehyds, und Verbesserung der Farbzahl der so hergestellten Glyoxallösungen. Auf einfachem Wege kann der Gehalt der Nebenstoffe der sich ergebenden Glyoxallösung verringert bzw. die Gesamtmenge an einem Nebenstoff, z.B. an Formaldehyd, abgetrennt werden. Die Farbzahl der Glyoxallösungen genügt auch den in diesem Zusammenhang hohen Anforderungen bei Anwendungen in der Textil-, Papier- und Lederindustrie, ohne daß eine besondere Reinigungsstufe, z.B. mit Ionenaustauschern, notwendig ist. Zersetzung der heißen Reaktionsgase und Verstopfungen der Rohrverbindungen und Anlagen bei der Abkühlung werden im Vergleich vermieden bzw. entscheidend verringert. Darüber hinaus bietet das Verfahren die Möglichkeit, den Formaldehyd auf einfachem Wege aus der Gasphase zu

entfernen, so daß eine aufwendige Reinigung der Glyoxallösungen entfällt. Im Zusammenhang mit der Arbeit in Ind. and Chem. Eng. liefert das erfindungsgemäße Verfahren schon mit einer einzigen Kondensationskolonne eine 40-gewichtsprozentige Glyoxallösung mit wesentlich geringerem Gehalt an Formaldehyd, Glykol, Glykolsäure und Ameisensäure. Alle diese vorteilhaften Ergebnisse sind im Hinblick auf den Stand der Technik überraschend. Insbesondere konnte nicht erwartet werden, daß die Farbzahl der hergestellten Glyoxallösungen als Maß der Verunreinigungen, die Zersetzung der heißen Gase während der Abkühlung und die Bildung von Crackprodukten allein durch die erfindungsgemäßen Abkühlungsbedingungen wesentlich verringert werden konnte. Denn es ist in Ind. and Chem. Eng. (loc. cit.) beschrieben, daß Glyoxal dazu neigt, in der Nähe seines Taupunktes mit geringen Wassermengen hornartige Polymere zu bilden. Auch war überraschend, daß nach dem erfindungsgemäßen Verfahren eine hohe Ausbeute und Reinheit des Endstoffs zu erzielen ist, obwohl wesentlich heißere Reaktionsgase innerhalb sehr kurzer Zeit abgekühlt werden müssen.

Ebenfalls war nicht zu erwarten, daß Glyoxal bei deutlich höheren Temperaturen als die in Beispiel 6 der Arbeit in Ind. and Chem. Eng. (loc. cit.) angegebenen vollständig aus den Reaktionsgasen auskondensiert werden kann. In diesem Beispiel wird nämlich angegeben, daß bei einer Abkühlung auf 50 °C im wesentlichen nicht umgesetztes Äthylenglykol kondensiert, das zum Teil mit Glyoxal das Nebenprodukt 2,3-Dihydroxydioxan bildet. Erst eine weitere Abkühlung der Gase auf 15 °C läßt Glyoxal, Wasser und Formaldehyd kondensieren. Bei dem erfindungsgemäßen Verfahren wird überraschend die unerwünschte Nebenproduktbildung von 2,3-Dihydroxydioxan nicht beobachtet.

Für das Verfahren geeignete Ausgangsstoffe sind reines Äthylenglykol, technisches Äthylenglykol bzw. Rohäthylenglykol oder vorteilhaft deren Mischungen mit Wasser ; die Konzentration der wäßrigen Gemische kann zweckmäßig zwischen 30 und 70 Gewichtsprozent, vorzugsweise zwischen 45 und 55 Gewichtsprozent Äthylenglykol schwanken. Vorteilhaft verwendet man 49,5- bis 50,5-gewichtsprozentige Lösungen bzw. Mischungen der beiden Komponenten in gleichen Gewichtsanteilen. Das Äthylenglykol wird in Dampfform, vorteilhaft im Gemisch mit Wasserdampf und gegebenenfalls mit Inertgas, dem Reaktionsraum zugeführt. Zweckmäßig verwendet man zusätzliches Inertgas. Als unter den Reaktionsbedingungen inerte Gase (Inertgas) werden zweckmäßig Edelgase wie Xenon, Argon, Neon, Helium ; Alkane wie Methan, Äthan, Propan, 2,2-Dimethylpropan, Butan, Pentan, Isobutan ; gasförmige organische Verbindungen anorganischer Elemente wie Tetramethylsilan ; Äther wie Dimethyläther, Methyläthyläther ; bevorzugt Stickstoff, Kohlenmonoxid und/oder Kohlendioxid ; und entsprechende Gemische verwendet. Das Inertgas kann für sich allein oder im Gemisch mit Wasserdampf und/oder Glykoldampf oder vorteilhaft im Gemisch mit Luft verwendet werden. Das Molverhältnis von Inertgas zu Sauerstoff beträgt in der Regel mindestens 4,4, zweckmäßig 4,4 bis 20, vorteilhaft 6 bis 10 zu 1. Die Angaben bezüglich Inertgas beziehen sich hier stets auf die Gesamtmenge, d. h. einschließlich des Inertgasanteils der bevorzugt verwendeten Luft. Gegebenenfalls kann auch das Abgas der Reaktion selbst als Inertgas verwendet werden, da es in der Regel neben den Inertgasen Stickstoff, Kohlenmonoxid, Kohlendioxid, Wasserstoff und Wasserdampf nur noch Reste unumgesetzten Ausgangsstoffs enthält, der so wiederverwertet wird.

Als oxidierendes Agens lassen sich sowohl der reine Sauerstoff als auch freien Sauerstoff enthaltende Gase, insbesondere Luft, verwenden. Sauerstoff, in der Regel in Gestalt von Luft, und Äthylenglykol werden zweckmäßig im Molverhältnis von 0,5 bis 1,2, insbesondere von 0,7 bis 1 Mol Sauerstoff je Mol Äthylenglykol angewandt. Vorzugsweise beträgt die Gesamtmenge an Wasserdampf nicht mehr als 5, vorteilhaft von 1 bis 4 Mol je Mol Äthylenglykol. Die Luft und gegebenenfalls das Inertgas können direkt in die Verdampfung des Äthylenglykols, zweckmäßig in die siedende Äthylenglykol/Wasser-Mischung, oder an einer beliebigen Stelle vor dem Katalysator eingeleitet werden. Die Verweilzeit im Reaktionsraum beträgt zweckmäßig höchstens 0,1, vorteilhaft von 0,000 5 bis 0,05, zweckmäßig von 0,001 bis 0,03, bevorzugt 0,001 bis 0,021 Sekunden.

Die Gesamtschichtdicke des Katalysators beträgt zweckmäßig 15 bis 50, vorzugsweise 20 bis 30 Millimeter. Die Umsetzung kann mit in einem weiten Bereich beliebigen Silberkatalysatoren nach einem der bekannten Verfahren durchgeführt werden. Vorteilhaft setzt man aber in Gegenwart von Silberkristallen mit einer Korngröße von 0,1 bis 2,5 Millimeter um. Die Katalysatorteilchen in Gestalt von Silberkristallen befinden sich vorteilhaft im Katalysator des üblicherweise vertikal aufgestellten Reaktors in einer Schicht oder je nach Korngröße in einem oberen oder unteren Teil der Gesamtschicht oder je nach Korngröße in einem oberen, mittleren oder unteren Teil der Gesamtschicht angeordnet. Das gesamte Katalysatorbett liegt zweckmäßig auf einem Netz aus Silber oder Edelstahl (vorgeglüht). Bei großen Reaktoren mit einem Durchmesser von mehr als 15 cm wird das Netz zweckmäßig vor Einbau gewellt. Das Netz liegt vorteilhaft auf einem Lochboden. Unmittelbar unter dem Lochboden befindet sich zweckmäßig die erfindungsgemäße Kondensationsanlage. Das Ausgangsgemisch aus Äthylenglykoldampf und Sauerstoff bzw. Luft und gegebenenfalls Wasserdampf und Inertgas wird im allgemeinen von oben nach unten geführt, so daß die obere Schicht (obere Schichten) gleichzeitig den dem Ausgangsgemisch zugewandten Teil bedeutet. Bei Reaktoren anderer Bauart oder anderer Führung des Ausgangsgemisches gelten sinngemäß alle Angaben der Beschreibung über oberen (unteren) Teil des Katalysators für den entsprechenden, dem Ausgangsgemisch (dem abgeführten Reaktionsgemisch) zugewandten Teil, z.B. bei horizontal angeordneten Reaktoren für den vorderen (hinteren) Teil des Katalysators. Hat der Katalysator nur eine Schicht, so enthält diese zweckmäßig Silberkristalle mit einer

Korngröße von 0,1 bis 2,5, vorteilhaft 0,1 bis 2, vorzugsweise 0,2 bis 1 Millimeter. Bei einem 2-Schichten-Katalysator enthält zweckmäßig die obere Teilschicht 20 bis 60, vorzugsweise von 25 bis 50 Gewichtsprozent des Katalysators mit Teilchen der Korngröße 0,1 bis 0,75 Millimeter, die untere Teilschicht 40 bis 80, vorzugsweise von 50 bis 75 Gewichtsprozent des Katalysators mit Teilchen der Korngröße 0,75 bis 2,5, vorteilhaft von 0,75 bis 1 Millimeter.

Bei einem 3-Schichten-Katalysator befinden sich im unteren Teil zweckmäßig 72,5 bis 89, vorzugsweise 77,5 bis 82,5 Gewichtsprozent aller Katalysatorteilchen, im mittleren Teil 2,5 bis 7,5, vorzugsweise 4,5 bis 6,5 Gewichtsprozent aller Katalysatorteilchen, im oberen Teil 8,5 bis 20, vorzugsweise 13 bis 16 Gewichtsprozent aller Katalysatorteilchen. Die Teilchen des unteren Schichtteils haben vorteilhaft Korngrößen von 1 bis 2,5, die des mittleren Schichtteils von 0,75 bis 1, die des oberen Schichtteils 0,1 bis 0,75 Millimeter.

Zweckmäßig belastet man den Katalysator mit 0,2 bis 3 t, insbesondere 0,3 bis 1 t Äthylenglykol je $m^2$ Katalysatorbettquerschnitt und Stunde. Zur großtechnischen Ausführung verwendet man bevorzugt Katalysatorbettdurchmesser von mindestens 0,2, zweckmäßig 0,5 bis 2 Meter.

Für die Oxidation leitet man vorteilhaft ein Gasgemisch aus Äthylenglykoldampf, Luft, Inertgas und zweckmäßig Wasserdampf in vorgenannten Mengen bei Temperaturen von 450 bis 710 °C, vorzugsweise von 550 bis 660 °C, durch den Silberkatalysator. Das Verfahren wird im allgemeinen bei Drücken zwischen 0,8 und 2 bar, vorzugsweise zwischen 0,8 und 1,8 bar, insbesondere 1,05 bis 1,5 bar, kontinuierlich durchgeführt.

Gegebenenfalls kann man auch Hemmstoffe dem Ausgangsgemisch, zweckmäßig in den Dampf/Gas-Strom der übrigen Komponenten vor dem Katalysator, zugeben. Vorteilhaft sind Halogenkohlenwasserstoffe, insbesondere Brom- und Chlorkohlenwasserstoffe, z.B. Tetrachloräthylen, 1,1,2,2- oder 1,1,1,2-Tetrachloräthan, Amylchlorid, Cyclohexylchlorid, 1,2-Dichlorpropan, Methylenchlorid, Dichlorbutan, Isopropylbromid, n-Propylbromid, Butylbromid, Chloroform, Bromoform, Äthyljodid, Propyljodid, Chlornaphthalin, Dichlornaphthalin, Tetrachlorkohlenstoff, 1,1,1- oder 1,1,2-Trichloräthan, Trichloräthylen, Pentachloräthan, 1,2-Dichloräthan, 1,1-Dichloräthan, n-Propylchlorid, 1,2-cis-Dichloräthylen, n-Butylchlorid, 2-, 3- und iso-Butylchlorid, Chlorbenzol, Fluorbenzol, Brombenzol, Jodbenzol, o-, p- und m-Dichlorbenzol, o-, p-, m-Dibrombenzol, o-, m-, p-Chlortoluol, 1,2,4-Trichlorbenzol, 1,10-Dibromdekan, 1,4-Dibrombutan ; und entsprechende Gemische. Bevorzugte Hemmstoffe sind insbesondere HCl, $Cl_2$, Bromoform. Zweckmäßig verwendet man den Hemmstoff in einer Menge von 0,01 bis 0,8 Gewichtsprozent, vorzugsweise von 0,05 bis 0,5 Gewichtsprozent, bezogen auf Äthylenglykol.

Im folgenden wird das Behandeln der heißen Reaktionsgase mit einer Flüssigkeit in feiner Verteilung zum Zwecke der Abkühlung der Gase und vollständiger oder teilweiser Kondensation kondensierbarer Anteile als Quenchen, der Vorgang selbst als der Quench definiert. Das aus dem Katalysator bzw. dem Boden der letzten Katalysatorschicht austretende dampf/gasförmige Reaktionsgemisch (Reaktionsgase) enthalten zweckmäßig 40 bis 85 Gewichtsprozent Stickstoff, 0,01 bis 1 Gewichtsprozent Sauerstoff, 0,5 bis 4 Gewichtsprozent Kohlenmonoxid, 1 bis 12 Gewichtsprozent Kohlendioxid, 3 bis 20 Gewichtsprozent Glyoxal, 0,01 bis 1 Gewichtsprozent unumgesetztes Äthylenglykol, 0,01 bis 4 Gewichtsprozent Glykolaldehyd, 0,1 bis 6 Gewichtsprozent Formaldehyd, 10 bis 40 Gewichtsprozent Wasser, 0,01 bis 1 Gewichtsprozent Glykolsäure, 0,01 bis 1 Gewichtsprozent Glyoxylsäure, 0,01 bis 1 Gewichtsprozent Ameisensäure, 0,001 bis 0,1 Gewichtsprozent 2-Hydroxymethyldioxolan, 0,1 bis 2 Gewichtsprozent Wasserstoff.

Die Strömungsgeschwindigkeit der Reaktionsgase beträgt vorteilhaft 0,1 bis 10, insbesondere 0,1 bis 5 Meter pro Sekunde. Die Verweilzeit der Reaktionsgase zwischen Katalysatorraum und Quenchraum beträgt höchstens eine Sekunde, zweckmäßig höchstens 0,1 Sekunden, vorteilhaft 0,001 bis 0,05, insbesondere 0,001 bis 0,01 Sekunden. Die Verweilzeit im Quenchraum beträgt höchstens eine Sekunde, vorteilhaft 0,01 bis 0,5, insbesondere 0,04 bis 0,2 Sekunden. Der Quenchraum wird als Zone der Behandlung mit der Quenchflüssigkeit definiert.

Der Quench wird mit der Quenchflüssigkeit, d.h. Wasser oder bevorzugt wäßriger Glyoxallösung, durchgeführt. Gegebenenfalls kann die Quenchflüssigkeit noch andere Stoffe, zweckmäßig Ausgangs- oder Nebenstoffe der Glyoxalsynthese enthalten, z.B. Äthylenglykol, Glykolaldehyd, Glykolsäure, Glyoxylsäure, Formaldehyd, Ameisensäure, 2-Hydroxymethyldioxolan. Zweckmäßig werden Glyoxallösungen, wie sie bei der Herstellung des Glyoxals anfallen, verwendet. Sie können 0,01 bis 5, insbesondere 0,01 bis 3 Gewichtsprozent Äthylenglykol, 0,1 bis 20, insbesondere 0,1 bis 10 Gewichtsprozent Glykolaldehyd, 0,01 bis 5, insbesondere 0,01 bis 3 Gewichtsprozent Glykolsäure, 0,01 bis 5, insbesondere 0,01 bis 3 Gewichtsprozent Glyoxylsäure, 0,1 bis 10, insbesondere 0,05 bis 5 Gewichtsprozent Formaldehyd, 0,01 bis 5, insbesondere 0,01 bis 3 Gewichtsprozent Ameisensäure, 0,05 bis 0,5, insbesondere 0,05 bis 0,2 Gewichtsprozent 2-Hydroxymethyldioxolan, zweckmäßig enthalten. Die Temperatur der Quenchflüssigkeit beträgt vorteilhaft 10 bis 120, insbesondere 30 bis 110 °C. Die Quenchflüssigkeit wird, gegebenenfalls nach Abkühlung bzw. Erhitzung, einer Zerteilungsvorrichtung zugeführt. Als Zerteilungsvorrichtungen kommen vorteilhaft Einrichtungen in Frage, die als Zerteilungsorgan Mischer, Zerstäuber und bevorzugt Düsen besitzen, z.B. Injektormischer, Mischkammern oder Mischstrecken mit Injektoren, Strahlmischer, Anlagen mit Drallkammerdüsen, Exzenterdüsen, Bündeldüsen, Zentrifugal-Druckdüsen, Schlitzdüsen, Flachstradhldüsen, Hohldüsen, Spiraldüsen und insbesondere Zerstaubungsdüsen, Zweistoffdüsen, Rotationszerstäuber, Pralldüsen, Vollkegeldüsen, rotierende

Zerstäuberscheiben mit Flügeln, rotierende Zerstäuberscheiben mit Düsen, Schlick-Düsen, Siccatom-Düsen, Hohlkegeldüse. Es können als Zerteilungsvorrichtung eine oder bevorzugt mehrere, zweckmäßig 4 bis 20 vorgenannte Düsen je Meter durchschnittlichen Durchmesser des Quenchraumes verwendet werden. Die in den Eingangsteil der Zerteilungsvorrichtung, z.B. der Düsenkammer, eintretende Quenchflüssigkeit wird im Zerteilungsteil, z.B. in der Düse, zu Tröpfchen von einem durchschnittlichen Durchmesser von 1 bis 2 000, vorzugsweise 1 bis 200 Mikrometern zerteilt. Die so gebildeten Tröpfchen treffen hinter der Zerteilung im Ausgangsteil der Zerteilungsvorrichtung, z.B. hinter der Düse, auf die Reaktionsgase.

Zweckmäßig liegen die Zerteilungsvorrichtungen, in der Regel Düsen, direkt hinter bzw. unter dem Ausgang des Katalysatorraums (Reaktionsraumes) bzw. der letzten Katalysatorschicht, vorteilhaft am Ausgang oder insbesondere hinter bzw. unter dem Reaktorausgang. Bevorzugt liegen die Düsen am Kopf eines Waschturms oder zweckmäßig einer Quenchkolonne, z.B. Füllkörper- oder Bodenkolonne, und der Ausgang des Reaktors liegt zweckmäßig über dem Waschturm bzw. der Quenchkolonne, so daß die Reaktionsgase nach Austritt aus dem Reaktionsraum sofort in den Quenchraum eintreten. Die Düsen können beliebig im Quenchraum verteilt sein, z.B. als Bündel in der Mitte des Quenchraumquerschnitts (Kolonnenquerschnitts) zusammengefaßt oder mit gleichen Abständen voneinander bzw. von der Wand verteilt. Vorteilhaft liegen sie ringförmig an oder in der Nähe der Wand des Quenchraumes (bzw. Kolonne), jede in demselben Abstand von ihren beiden Nachbardüsen entfernt, verteilt. In einer bevorzugten Anordnung bildet man mehrere solcher Ringe hintereinander, jeder Ring an der oder in der Nähe der Kolonnenwand, vorteilhaft 4 bis 20, insbesondere 8 bis 16 Düsen je Ring und je Meter durchschnittlichen Durchmesser des Quenchraums und 2 bis 5, vorteilhaft 2 bis 3 Ringe hintereinander. Die Düsen jedes Ringes stehen zweckmäßig zu denen des vorhergehenden und/oder nachfolgenden Ringes auf Lücke. Zweckmäßig wählt man einen rohrförmigen Quenchraum (Kolonne) mit einem Verhältnis der Länge zum Durchmesser des Quenchraumes wie 0,1 bis 3, insbesondere 0,2 bis 1. Im allgemeinen liegen die Düsenringe soweit voneinander entfernt, daß der gesamte Quenchraum einschließlich des Raumes oberhalb der Quenchraumwand (Kolonnenwand) mit dem Gas/Dampf/Flüssigkeitsnebel des feinverteilten Gemisches aus Tröpfchen und gas- bzw. dampfförmigen Anteilen gleichmäßig gefüllt ist.

Die Düsen sind in der Regel so angeordnet, daß die aus den Düsen austretende Flüssigkeit den ganzen Raum bestreicht bzw. an den Wänden des Quenchraumes zurückgeworfen wird ; auf diese Weise stellt der Quenchraum eine Schicht von Flüssigkeits/Gasnebel dar, auf den die heißen Reaktionsgase treffen. Der Düsenaustritt liegt nicht parallel zur Stromachse der Reaktionsgase, sondern alle Düsenaustritte oder Düsenachsen bilden jeweils einen Winkel mit der Stromachse, so daß der Hauptteil aller von der Düse versprühten Tröpfchen, vorteilhaft 60 bis 100, insbesondere 60 bis 80 Gewichtsprozent der gesamten Quenchflüssigkeit mit einem Winkel zur Stromachse, vorteilhaft 15 bis 85, zweckmäßig 20 bis 85, vorzugsweise 25 bis 82, insbesondere 30 bis 80° zur Stromachse, auf den Strom der Reaktionsgase trifft. Durch die vorgenannte, bevorzugte Anordnung der Düsen im Ring und der Ringe an und in der Nähe der Kolonnenwand am Kopf der Quenchkolonne sowie durch bevorzugt unterschiedliche Winkeleinstellung der Düsen wird vorteilhaft eine gleichmäßige Verteilung des Flüssigkeits/Gasnebels innerhalb des Quenchraumes und über der Kolonnenwand gebildet. Bei der Winkelmessung wird 0 als parallel und 90 als senkrecht zur Stromachse definiert. Bevorzugt wird der Winkel so eingestellt, daß die Düsen die Quenchflüssigkeit mit vorgenanntem Winkel zur Stromachse im Gegenstrom zu den Reaktionsgasen sprühen, jedoch ist auch eine Versprühung der Quenchflüssigkeit im Gleichstrom, d.h. eine Einstellung der Düsenaustritte in Stromrichtung mit vorgenannten Winkeln zur Stromachse möglich. Zweckmäßig verwendet man 0,5 bis 60, vorzugsweise 1 bis 40 Kilogramm Wasser oder wäßrige Glyoxallösung je Kilogram Reaktionsgase. Vorteilhaft beträgt die Menge an Inertgas von 50 bis 95, insbesondere von 70 bis 95 Gewichtsprozent, bezogen auf das Gesamtgewicht der Reaktionsgase, die Verweilzeit der Reaktionsgase in dem Quenchraum 0,01 bis 0,5, insbesondere 0,04 bis 0,2 Sekunden, die Temperatur des aus dem Quenchraum austretenden Reaktionsgasanteils (Abgas) bis 150, insbesondere 25 bis 120 °C, ihre Strömungsgeschwindigkeit 0,1 bis 10, insbesondere 0,1 bis 5 Meter pro Sekunde. Der Quench wird kontinuierlich durchgeführt. Bei dem Quench wird stets Wasser und jeweils nach den Bedingungen Glyoxal, Glykol und/oder Glykolaldehyd kondensiert. Gewünschtenfalls befinden sich die Anteile an Formaldehyd, Glykolsäure, Glyoxylsäure, Ameisensäure und/oder 2-Hydroxymethyldioxolan in den Reaktionsgasen ganz oder teilweise im Kondensat des Quenchs.

Durch die Wahl vorgenannter Parameter, insbesondere der Art der Quenchflüssigkeit, der Temperatur und Menge der Quenchflüssigkeit, der Menge an Inertgas im Reaktionsgasstrom und der Verweilzeit der Gase im Quenchraum, können die kondensierbaren Anteile ganz oder teilweise im Quenchraum kondensiert werden. Je höher a) die Temperatur der Quenchflüssigkeit, zweckmäßig von 10 bis 120 °C, je geringer b) die Menge der Quenchflüssigkeit, zweckmäßig von 0,5 bis 60 Kilogramm Wasser oder wäßrige Glyoxallösung je Kilogramm Reaktionsgase, je höher c) die Inertgasmenge, zweckmäßig von 50 bis 95 Gewichtsprozent, bezogen auf die Gewichtsmenge der Reaktionsgase, desto niedriger die Menge an Kondensat. Verringert man in den vorgenannten, zweckmäßige Parameterbereichen den Wert a) der Temperatur der Quenchflüssigkeit von 120 bis 0 °C, so können, entsprechend sich steigernd, von 40 bis 100 Gewichtsprozent der Gesamtmenge an Glykolaldehyd in den Reaktionsgasen, entsprechend (jeweils bezogen auf die Gesamtmenge dieses Stoffes in den Reaktionsgasen) 80 bis 100 Gewichtsprozent Glykol,

5 bis 100 Gewichtsprozent Glyoxal, 0 bis 100 Gewichtsprozent Formaldehyd, 4 bis 100 Gewichtsprozent Wasser kondensiert werden. Steigert man entsprechend in den vorgenannten, zweckmäßigen Parameterbereichen der Wert von b) von 0,5 bis 60 Kilogramm Wasser je Kilogramm Reaktionsgase, so erhält man, entsprechend sich steigernd, im Kondensat (jeweils bezogen auf die Gesamtmenge dieses Stoffes in den Reaktionsgasen) 75 bis 100 Gewichtsprozent Glykolaldehyd, 80 bis 100 Gewichtsprozent Glykol, 5 bis 100 Gewichtsprozent Glyoxal, 0,5 bis 97 Gewichtsprozent Formaldehyd, 4 bis 95 Gewichtsprozent Wasser. Verringert man in den vorgenannten, zweckmäßigen Parameterbereichen den Wert von c) der Inertgasmenge von 95 bis 50 Gewichtsprozent, bezogen auf die Gewichtsmenge der Reaktionsgase, so erhält man, entsprechend sich steigernd, im Kondensat (jeweils bezogen auf die Gesamtmenge dieses Stoffes in den Reaktionsgasen) 80 bis 100 Gewichtsprozent Glykolaldehyd, 85 bis 100 Gewichtsprozent Glykol, 5 bis 100 Gewichtsprozent Glyoxal, 0 bis 97 Gewichtsprozent Formaldehyd, 4 bis 95 Gewichtsprozent Wasser. Vergrößert man d) die Verweilzeit der Reaktionsgase im Quenchraum, z.B. von 0,01 bis 0,7 Sekunden, so können, entsprechend sich steigernd (jeweils bezogen auf die Gesamtmenge dieses Stoffes in den Reaktionsgasen), von 50 bis 100 Gewichtsprozent Glykolaldehyd, von 70 bis 100 Gewichtsprozent Glykol, von 0 bis 100 Gewichtsprozent Glyoxal, von 0 bis 97 Gewichtsprozent Formaldehyd, von 4 bis 95 Gewichtsprozent Wasser kondensiert werden. Steigert man die Konzentration der wäßrigen Glyoxallösung von oberhalb 0 (Wasser) bis 65 Gewichtsprozent, so steigert sich entsprechend die Konzentration des Kondensates von 0 bis 65 Gewichtsprozent Glyoxal, von 0,01 bis 5 Gewichtsprozent Glykol, von 0,1 bis 10 Gewichtsprozent Formaldehyd, von 0,1 bis 20 Gewichtsprozent Glykolaldehyd im Kondensat. Beispielsweise ergeben sich im Kondensat, bezogen auf die Gesamtmenge des jeweiligen Stoffes in den Reaktionsgasen, die in den folgenden Tabellen aufgeführten Anteile unter den angegebenen Bedingungen. Ein bestimmtes Verhältnis der Anteile im Kondensat der Quenchkolonne kann so anhand einiger Versuche leicht festgelegt werden.

TABELLE

| Anteile in Gew.% im Kondensat | Temperatur der Quenchflüssigkeit in °C | Konzentration der Quenchflüssigkeit in Gew.% Glyoxal | Menge an Quenchflüssigkeit in kg je kg Reaktionsgas | Verweilzeit der Reaktionsgase im Quenchraum in Sekunden | Menge an Inertgas in Gew.%, bezogen auf die Reaktionsgase |
|---|---|---|---|---|---|
| 50 Glyoxal ; 90 Glykol ; 90 Glykolaldehyd ; 10 Wasser ; 2 $CH_2O$ | 92 | 21 | 20 | 0,08 | 85 |
| 20 Glyoxal ; 90 Glykol ; 80 Glykolaldehyd ; 5 Wasser ; 1 $CH_2O$ | 95 | 11 | 6 | 0,05 | 90 |
| 100 Glyoxal ; 100 Glykol ; 100 Glykolaldehyd ; 80 Wasser ; 95 $CH_2O$ | 25 | 24 | 33 | 0,12 | 70 |
| 80 Glyoxal ; 100 Glykol ; 95 Glykolaldehyd ; 15 Wasser ; 10 $CH_2O$ | 90 | 42 | 23 | 0,1 | 86 |
| 100 Glyoxal ; 100 Glykol ; 100 Glykolaldehyd ; 50 Wasser ; 60 $CH_2O$ | 70 | 50 | 50 | 0,09 | 92 |
| 90 Glyoxal ; 100 Glykol ; 98 Glykolaldehyd ; 30 Wasser ; 12 $CH_2O$ | 88 | 40 | 30 | 0,1 | 85 |
| 100 Glyoxal ; 100 Glykol ; 100 Glykolaldehyd ; 95 Wasser ; 97 $CH_2O$ | 10 | 23 | 60 | 0,2 | 50 |
| 70 Glyoxal ; 95 Glykol ; 92 Glykolaldehyd ; 10 Wasser ; 3 $CH_2O$ | 95 | 36 | 15 | 0,09 | 84 |

In der Regel verhalten sich bezüglich der Kondensation bei den vorgenannten Bedingungen die Glykolsäure ähnlich Glykol, die Glyoxylsäure ähnlich Glykol, die Ameisensäure ähnlich Wasser, das 2-Hydroxymethyldioxolan ähnlich Glykolaldehyd und können entsprechend den für die Vergleichsstoffe vorgenannten Bedingungen ganz oder teilweise kondensiert werden.

In den so erhaltenen, von 0 bis 65-gewichtsprozentigen Glyoxallösungen sind bei vorgenannten erfindungsgemäßen Bedingungen im allgemeinen 0,01 bis 5, insbesondere 0,01 bis 3 Gewichtsprozent Glykol, 0,1 bis 20, insbesondere 0,1 bis 10 Gewichtsprozent Glykolaldehyd, 0,1 bis 10, insbesondere 0,1 bis 5 Gewichtsprozent Formaldehyde, 0,01 bis 5, insbesondere 0,01 bis 3 Gewichtsprozent Glykolsäure, 0,01 bis 5, insbesondere 0,01 bis 3 Gewichtsprozent Glyoxylsäure, 0,01 bis 5, insbesondere 0,01 bis 3 Gewichtsprozent Ameisensäure, 0,05 bis 0,5, insbesondere 0,05 bis 0,2 Gewichtsprozent 2-Hydroxymethyldioxolan enthalten. Die Quenchflüssigkeit kann der vorgenannten Glyoxallösung direkt entnommen und gewünschtenfalls mit Wasser verdünnt werden ; ebenfalls können Glyoxallösungen aus einer anderen Herstellungsanlage, aber von zweckmäßig vorgenannter Zusammensetzung, als Quenchflüssigkeit verwendet werden.

In einer zweckmäßigen Ausführungsform des Verfahrens wird das nach Abtrennung des Kondensats verbleibende Abgas der Reaktion ganz oder in der Regel teilweise wieder zugeführt. Entsprechend kann die Kondensation auch so eingestellt werden, daß der gesamte Anteil an Formaldehyd im Abgas verbleibt und entfernt bzw. in einer weiteren Absorptionskolonne in Gestalt einer wäßrigen, 5- bis 60-gewichtsprozentigen Formaldehydlösung abgetrennt wird. Das Kondensat kann als gebrauchsfertige Glyoxallösung vollständig entnommen oder teilweise als Kühlflüssigkeit im Kreis geführt werden. Nach dem erfindungsgemäßen Verfahren kann so in mehrstufiger Kondensation gleichzeitig eine fast formaldehydfreie und eine mit Formaldehyd angereicherte Lösung hergestellt werden. Durch Wahl vorgenannter Parameter wird z.B. in der ersten Kolonne nur ein Teil des Glyoxals auskondensiert und in einer zweiten Kolonne das restliche Glyoxal zusammen mit dem Formaldehyd in wäßriger Lösung aufgefangen. Da industriell häufig einerseits eine formaldehydfreie und andererseits eine mit Formaldehyd angereicherte Glyoxallösung benötigt wird, ist es ein Vorteil des erfindungsgemäßen Verfahrens, daß diese verschiedenen Qualitätsansprüche in einer Anlage verwirklicht werden können. Es können im allgemeinen je nach den gewählten vorgenannten Bedingungen im Quenchraum und damit auch in der ersten Kolonne 0 bis 100 Gewichtsprozent Glyoxal, 70 bis 100 Gewichtsprozent Glykol, 50 bis 100 Gewichtsprozent Glykolaldehyd, 4 bis 100 Gewichtsprozent Wasser, 0 bis 100 Gewichtsprozent Ameisensäure, 50 bis 100 Gewichtsprozent 2-Hydroxymethyldioxolan, 70 bis 100 Gewichtsprozent Glyoxylsäure, bezogen auf den in den Reaktionsgasen ursprünglich enthaltenen Anteil des jeweiligen Stoffes, kondensiert und abgetrennt werden.

Im allgemeinen werden die wäßrigen Lösungen, zweckmäßig in 40-Gewichtsprozentiger Form, direkt weiterverwendet, gegebenenfalls kann man den Endstoff in üblicher Weise, z.B. durch Destillation mit wasserentziehenden Mitteln, isolieren.

Das nach dem Verfahren der Erfindung herstellbare Glyoxal ist Knitterfestmittel, Hilfsmittel zur Erhöhung der Reißfestigkeit und Elastizität von Faserstoffen, Gerbstoff, Härter in der photographischen Industrie und wertvoller Ausgangsstoff für die Herstellung von Kunstharzen, Textilhilfsmitteln, z.B. zur Verhinderung des Einlaufens nach dem Waschen, Papierhilfsmitteln, z.B. zur Erhöhung der Naßfestigkeit, und Kunststoffen. Bezüglich der Verwendung wird auf vorgenannte Veröffentlichungen und Ullmann (loc. cit.) verwiesen.

Die in den folgenden Beispielen angeführten Teile bedeuten Gewichtsteile.

Beispiel 1

(die Ziffern in Klammern beziehen sich auf Figur 1)

a) Man verwendet eine Anlage mit Äthylenglykol-Verdampfer (1) entsprechenden Zuführungen von Äthylenglykol (6), Wasser (7) und Luft bzw. Stickstoff (11) und einem senkrechten Rohrreaktor (2). Der Reaktor enthält an seinem Kopf die Zuführung (12) für das dampfförmige Ausgangsgemisch und die Reaktorhaube. Die Katalysatorschicht liegt unterhalb des Reaktorkopfes, weiter unten folgt der Quenchraum (10), der den Kopf einer Füllkörperkolonne (3) bildet. Das Kondensat wird teilweise als Quenchflüssigkeit verwendet. Die Quenchflüssigkeit wird mit einer Pumpe (4) über einen Wärmetauscher (5) und ein Düsensystem unmittelbar hinter der Katalysatorschicht in die heißen Gase gesprüht, die im Quenchraum auf 70 °C abgekühlt und teilweise kondensiert werden, so daß über die Leitung (8) eine 27,5-gewichtsprozentige Glyoxallösung abgenommen werden kann. Das Abgas entweicht über die Leitung (9).

In den Reaktor (2) wird ein Katalysator aus Silberkristallen (45 Teilen) folgender Zusammensetzung eingetragen :

| | Anteil am Katalysator (Gew.%) | Korngröße mm |
|---|---|---|
| Schicht 1 | 15 | 0,1-0,75 |
| Schicht 2 | 5 | 0,75-1 |
| Schicht 3 | 80 | 1-2,5 |

Die Höhe des Katalysators beträgt 30 mm. Dem Verdampfer (1) wird pro Stunde ein Gemisch von 246 Teilen Äthylenglykol, 246 Teilen Wasser, 456 Teilen Luft und 340 Teilen Stickstoff zugeführt und verdampft. Das dampfförmige Ausgangsgemisch wird durch den Katalysator geleitet und bei 597 °C und 1,12 bar umgesetzt. Die Verweilzeit im Katalysatorraum beträgt 0,01 Sekunden. Die in den Quenchraum eintretenden Reaktionsgase (stündlich 1 288 Teile) enthalten 53,7 Gewichtsprozent $N_2$, 0,2 Gewichtsprozent $O_2$, 1,6 Gewichtsprozent CO, 3,4 Gewichtsprozent $CO_2$, 10,7 Gewichtsprozent Glyoxal, 0,4 Gewichtsprozent Äthylenglykol, 26,5 Gewichtsprozent Wasser, 0,7 Gewichtsprozent Formaldehyd, 2 Gewichtsprozent Glykolaldehyd, 0,05 Gewichtsprozent Glykolsäure, 0,1 Gewichtsprozent Ameisensäure, 0,005 Gewichtsprozent 2-Hydroxymethyldioxolan, 0,04 Gewichtsprozent Glyoxylsäure, 0,3 Gewichtsprozent Wasserstoff.

Die Strömungsgeschwindigkeit der Reaktionsgase beträgt 2,6 Meter pro Sekunde, die Verweilzeit zwischen Katalysatorschicht und Quenchraum 0,004 Sekunden, die Temperatur der Reaktionsgase vor Eintritt in den Quenchraum 580 °C, die der Quenchflüssigkeit 70 °C, die Verweilzeit im Quenchraum, 0,11 Sekunden, die Temperatur der Gase nach Austritt aus dem Quenchraum 85 °C, die Menge der Quenchflüssigkeit 8 000 Teile pro Stunde.

Es werden 2 Ringe mit jeweils 6 auf Lücke angeordneten Düsen verwendet. Die Düsen eines Ringes befinden sich an der Kolonnenwand und sind symmetrisch angeordnet. Der Auftreffwinkel der Tröpfchen zur Stromachse aller Düsen ist unterschiedlich und beträgt 15 bis 75°, von 70 % der Tröpfchen 30 bis 75°. Die Tröpfchen haben einen durchschnittlichen Durchmesser von 200 Mikrometern. In Form einer 41-gewichtsprozentigen Glyoxallösung erhält man 337 Teile Lösung oder 138 Teile pro Stunde Glyoxal, entsprechend einer Ausbeute von 60 % der Theorie, bezogen auf verwendetes Glykol. Die Lebensdauer des Katalysators beträgt 90 Tage. Die Glyoxallösung (und somit die Quenchflüssigkeit) hat einen Gehalt von 1,6 Gewichtsprozent Äthylenglykol, 1,4 Gewichtsprozent Formaldehyd, 8,2 Gewichtsprozent Glykolaldehyd, 0,2 Gewichtsprozent Glykolsäure, 0,4 Gewichtsprozent Ameisensäure, 0,07 Gewichtsprozent 2-Hydroxymethyldioxolan, 0,2 Gewichtsprozent Glyoxylsäure. Der Umsatz beträgt 97,8 Prozent, die Raum-Zeit-Ausbeute 14,7 g Glyoxal pro $cm^3$ Katalysatorvolumen und Stunde. Farbzahl der Lösung nach dreitägigem Betrieb : 22 (die Farbzahl wird mittels der Platin-Cobalt-Skala nach ASTM D 1209-69 bestimmt). Auch während einer Betriebszeit von 30 Tagen bilden sich keine festen Rückstände.

b) Vergleich :

Die Anlage enthält statt Quenchraum (10) und Füllkörperkolonne (3) zwei hinter den Reaktor geschalteten, mit Wasser gekühlten Kondensationskolonnen. Die Umsetzung wird analog Beispiel 1 durchgeführt. Die Reaktionsgase werden in der ersten Kühlzone auf 200 °C, in der 2. Kühlzone auf 10 °C abgekühlt, wobei alle kondensierbaren Dämpfe in flüssiger Form anfallen. In Form einer 25,3-gewichtsprozentigen Glyoxallösung erhält man 498 Teile Lösung oder 126 Teile pro Stunde Glyoxal, entsprechend einer Ausbeute von 55 % der Theorie. Die Glyoxallösung hat einen Gehalt von einem Gewichtsprozent Glykol und 1,8 Gewichtsprozent Formaldehyd, 5,2 Gewichtsprozent Glykolaldehyd, 0,3 Gewichtsprozent Glykolsäure, 0,4 Gewichtsprozent Ameisensäure, 0,07 Gewichtsprozent 2-Hydroxymethyldioxolan, 0,3 Gewichtsprozent Glyoxylsäure. Der Umsatz beträgt 97,8 Prozent, die Raum-Zeit-Ausbeute 13,5 Gramm Glyoxal pro $cm^3$ Katalysatorvolumen und Stunde. Farbzahl der Lösung nach dreitägigem Betrieb 500 (die Farbzahl wird mittels der Platin-Cobalt-Skala nach ASTM D 1209-69 bestimmt). Am Ende einer Betriebszeit von 3 Tagen werden 12 Teile Rückstände durch Crackung im ersten Kühler festgestellt.

c) Vergleich (Figur 2) :

Analog Beispiel 1a) wird die Reaktion durchgeführt, anstelle des Quenchraumes wird ein an dem senkrechten Rohrreaktor (2) angeflanschter Rohrbündelwärmetauscher (13) verwendet, in dem die heißen Reaktionsgase auf 190 °C abgekühlt werden. Die kondensierbaren Dämpfe werden in der sich anschließenden Absorptionskolonne (14) ausgewaschen. Als Kühl- und Waschflüssigkeit dient die anfallende 21,6-gewichtsprozentige Glyoxallösung. Ein Teil wird über die Leitung (15) der Anlage entnommen, der Rest wird mit einer Pumpe (16) durch den Wärmetauscher (17), in dem die Waschflüssigkeit (20 °C) gekühlt wird, im Kreis gefahren. Die Waschflüssigkeit (8 000 Teile/h) trifft im Gegenstrom aus 6 Düsen (20) frontal auf die Reaktionsgase, der Winkel zur Stromachse ist 0. Die Verweilzeit zwischen Katalysator und den Düsen 1,2 Sekunden, die Temperatur der Gase vor der Wäsche 170 °C. Eventuell im Rohrbündelwärmetauscher (13) anfallendes Kondensat sammelt sich in der Vorlage (18) und wird über die Leitung (19) aus der Analge abgezogen. Das Abgas entweicht über die Leitung (9). Die über die Leitungen (6), (11), (12) und (7) zugeführten Zuläufe und die Katalysatorschichtung und anordnung im Reaktor (2) werden analog Beispiel 1 gewählt.

In Form einer 21,6-gewichtsprozentigen Glyoxallösung erhält man 508 Teile Lösung oder 110 Teile pro Stunde Glyoxal, entsprechend einer Ausbeute von 48 % der Theorie. Die Glyoxallösung hat einen Gehalt von einem Gewichtsprozent Glykol und 3,5 Gewichtsprozent Formaldehyd, 5,1 Gewichtsprozent Glykolaldehyd, 0,5 Gewichtsprozent Glykolsäure, 0,7 Gewichtsprozent Ameisensäure, 0,07 Gewichtsprozent 2-Hydroxymethyldioxolan, 0,6 Gewichtsprozent Glyoxylsäure. Farbzahl der Lösung nach 10 Tagen Betriebsdauer : 800 (die Farbzahl wird mittels der Platin-Cobalt-Skala nach ASTM D 1209-69 bestimmt). Am Ende der Betriebszeit von 10 Tagen sind die Rohre des Wärmetauschers (13) nach dem Reaktor mit abgelagerten Crackprodukten verstopft.

Beispiel 2

Es wird die gleiche Anlage wie in Beispiel 1 verwendet. Die Umsetzung wird analog Beispiel 1 durchgeführt, wobei lediglich die Quenchbedingungen verändert werden. Es werden 2 Ringe mit jeweils 6 auf Lücke angeordneten düsen verwendet. Die Düsen eines Ringes befinden sich an der Kolonnenwand und sin symmetrisch angeordnet. Der Auftreffwinkel der Tröpfchen zur Stromachse aller Düsen ist unterschiedlich und beträgt 15 bis 75°, von 70 % der Tröpfchen 30 bis 75°. Die Tröpfchen haben einen durchschnittlichen Durchmesser von 200 Mikrometern. Die Strömungsgeschwindigkeit der Reaktionsgase beträgt 2,6 Meter pro Sekunde, die Verweilzeit zwischen Katalysatorschicht und Quenchraum 0,004 Sekunden, die Temperatur der Reaktionsgase vor Eintritt in den Quenchraum 580 °C, die der Quenchflüssigkeit 65 °C, die Verweilzeit im Quenchraum 0,08 Sekunden, die Temperatur der Gase nach Austritt aus dem Quenchraum 85 °C, die Menge der Quenchflüssigkeit 7 000 Teile pro Stunde.

In Form einer 40-gewichtsprozentigen Glyoxallösung erhält man 345 Teile Lösung oder 138 Teile pro Stunde Glyoxal, entsprechend einer Ausbeute von 60 % der Theorie. Die Lebensdauer des Katalysators beträgt 90 Tage. Die Glyoxallösung (und somit die Quenchflüssigkeit) hat einen Gehalt von 1,5 Gewichtsprozent Glykol, 0,9 Gewichtsprozent Formaldehyd, 8,1 Gewichtsprozent Glykolaldehyd, 0,2 Gewichtsprozent Glykolsäure, 0,4 Gewichtsprozent Ameisensäure, 0,07 Gewichtsprozent 2-Hydroxymethyldioxolan, 0,2 Gewichtsprozent Glyoxylsäure. Der Umsatz beträgt 97,8 Prozent, die Raum-Zeit-Ausbeute 14,6 Gramm Glyoxal pro $cm^3$ Katalysatorvolumen und Stunde. Farbzahl der Lösung nach dreitägigem Betrieb : 15 (die Farbzahl wird mittels der Platin-Cobalt-Skala nach ASTM D 1209-69 bestimmt). Auch während einer Betriebszeit von 30 Tagen bilden sich keine festen Rückstände.

Beispiel 3

Es wird die gleiche Anlage wie in Beispiel 1 verwendet. Die Umsetzung wird analog Beispiel 1 durchgeführt. Die Stickstoffzuführung über Leitung (11) wird auf 1 400 Teile pro Stunde erhöht, die Luftzuführung wird auf 520 Teile pro Stunde erhöht. Stündlich werden der Quenchflüssigkeit zusätzlich 250 Teile Wasser über die Leitung (21) zugegeben. Die in den Quenchraum eintretenden Reaktionsgase (stündlich 2 412 Teile) enthalten 74,6 Gewichtsprozent $N_2$, 0,1 Gewichtsprozent $O_2$, 0,9 Gewichtsprozent CO, 1,9 Gewichtsprozent $CO_2$, 5,9 Gewichtsprozent Glyoxal, 0,2 Gewichtsprozent Äthylenglykol, 14,3 Gewichtsprozent Wasser, 0,4 Gewichtsprozent Formaldehyd, 1,1 Gewichtsprozent Glykolaldehyd, 0,04 Gewichtsprozent Glykolsäure, 0,05 Gewichtsprozent Ameisensäure, 0,005 Gewichtsprozent 2-Hydroxymethyldioxolan, 0,05 Gewichtsprozent Glyoxalsäure, 0,1 Gewichtsprozent Wasserstoff. Die Strömungsgeschwindigkeit der Reaktionsgase beträgt 4,7 Meter pro Sekunde, die Verweilzeit zwischen Katalysatorschicht und Quenchraum 0,002 Sekunden, die Temperatur der Reaktionsgase vor Eintritt in den Quenchraum 580 °C, die der Quenchflüssigkeit 60 °C, die Verweilzeit im Quenchraum 0,06 Sekunden, die Temperatur des Gase nach Austritt aus dem Quenchraum 65 °C, die Menge der Quenchflüssigkeit 8 000 Teile pro Stunde.

In Form einer 40-gewichtsprozentigen Glyoxallösung erhält man 358 Teile Lösung oder 143 Teile pro Stunde Glyoxal, entsprechend einer Ausbeute von 62 % der Theorie. Die Lebensdauer des Katalysators beträgt 90 Tage. Die Glyoxallösung hat einen Gehalt von 1,5 Gewichtsprozent Glykol und 0,2 Gewichtsprozent Formaldehyd, 6,9 Gewichtsprozent Glykolaldehyd, 0,2 Gewichtsprozent Glykolsäure, 0,4 Gewichtsprozent Ameisensäure, 0,07 Gewichtsprozent 2-Hydroxymethyldioxolan, 0,2 Gewichtsprozent Glyoxylsäure. Die Quenchflüssigkeit hat einen Gehalt von 37,7 Gewichtsprozent Glyoxal, 1,4 Gewichtsprozent Glykol, 0,2 Gewichtsprozent Formaldehyd, 6,5 Gewichtsprozent Glykolaldehyd, 0,2 Gewichtsprozent Glykolsäure, 0,4 Gewichtsprozent Ameisensäure, 0,07 Gewichtsprozent 2-Hydroxymethyldioxolan, 0,2 Gewichtsprozent Glyoxylsäure. Farbzahl der Lösung nach dreitägigem Betrieb : 14 (die Farbzahl wird mittels der Platin-Cobalt-Skala nach ASTM D 1209-69 bestimmt). Auch während einer Betriebszeit von 30 Tagen bilden sich keine festen Rückstände.

Beispiel 4 (Figur 3)

Die Reaktion wird analog Beispiel 1 durchgeführt. Die Anlage entspricht der Anlage von Beispiel 1 (Figur 1), aber neben dem Quenchraum (10) un der Füllkörperkolonne (3) einen zusätzlichen Quenchraum (22) mit einer Füllkörperkolonne (23), die dem ersten Quench unmittelbar nachgeschaltet sind. Die Quenchflüssigkeit im zweiten Kreislauf wird mit einer Pumpe (24) über einen Wärmetauscher (25) und ein Düsensystem in die über die Leitung (26) eintretenden Gase unter einem Wirbel eingesprüht. Die Gase werden im Quenchraum abgekühlt, kondensiert und man zieht über die Leitung (27) eine 40-gewichtsprozentige Glyoxallösung ab. Das Kondensat wird teilweise als Quenchflüssigkeit verwendet. Das Abgas entweicht über die Leitung (9). Das Düsensystem, der Durchmesser und die Auftreffwinkel der Tröpfchen entsprechen Beispiel 1.

Stündlich werden der Quenchflüssigkeit zusätzlich 400 Teile Wasser über die Leitung (21) zugegeben. Die in den Quenchraum eintretenden Reaktionsgase (stündlich 2 412 Teile) enthalten 74,6 Gewichtsprozent $N_2$, 0,1 Gewichtsprozent $O_2$, 0,9 Gewichtsprozent CO, 1,9 Gewichtsprozent $CO_2$, 5,9 Gewichtsprozent Glyoxal, 0,2 Gewichtsprozent Äthylenglykol, 14,6 Gewichtsprozent Wasser, 0,4 Gewichtsprozent Formaldehyd, 1,1 Gewichtsprozent Glykolaldehyd, 0,04 Gewichtsprozent Glykolsäure, 0,05 Gewichtsprozent Ameisensäure, 0,005 Gewichtsprozent 2-Hydroxymethyldioxolan, 0,05 Gewichtsprozent Glyoxylsäure, 0,2 Gewichtsprozent Wasserstoff. Die Strömungsgeschwindigkeit der Reaktionsgase beträgt 4,7 Meter pro Sekunde, die Verweilzeit zwischen Katalysatorschicht und Quenchraum 0,002 Sekunden, die Temperatur der Reaktionsgase vor Eintritt in den Quenchraum (10) 580 °C, die der Quenchflüssigkeit 75 °C, die Verweilzeit im Quenchraum (10) 0,05 Sekunden, die Temperatur des Gase nach Austritt aus der Füllkörperkolonne (3) 85 °C, die Menge der Quenchflüssigkeit im ersten Kühlkreis 5 000 Teile pro Stunde.

In Form einer 40-gewichtsprozentigen Glyoxallösung werden über die Leitung (8) 298 Teile Lösung oder 119 Teile pro Stunde Glyoxal entnommen, entsprechend einer ersten Teilausbeute von 51,7 % der Theorie. Die Glyoxallösung hat einen Gehalt von 1,8 Gewichtsprozent Glykol und 0,1 Gewichtsprozent Formaldehyd, 7,8 Gewichtsprozent Glykolaldehyd, 0,2 Gewichtsprozent Glykolsäure, 0,1 Gewichtsprozent Ameisensäure, 0,06 Gewichtsprozent 2-Hydroxymethyldioxolan, 0,2 Gewichtsprozent Glyoxylsäure. Die Quenchflüssigkeit hat einen Gehalt von 34,5 Gewichtsprozent Glyoxal, 1,6 Gewichtsprozent Glykol, 0,08 Gewichtsprozent Formaldehyd, 6,7 Gewichtsprozent Glykolaldehyd, 0,2 Gewichtsprozent Glykolsäure, 0,05 Gewichtsprozent Ameisensäure, 0,005 Gewichtsprozent 2-Hydroxymethyldioxolan, 0,2 Gewichtsprozent Glyoxylsäure. Farbzahl der Lösung nach dreitägigem Betrieb : 22 (die Farbzahl wird mittels der Platin-Cobalt-Skala nach ASTM D 1209-69 bestimmt). Auch während einer Betriebszeit von 30 Tagen bilden sich keine festen Rückstände. Die Temperatur der Reaktionsgase vor Eintritt in den zweiten Quenchraum (22) beträgt 85 °C, die der zweiten Quenchflüssigkeit 73 °C, die Verweilzeit im Quenchraum (22) 0,05 Sekunden, die Temperatur der Gase nach Austritt aus dem Quenchraum (22) 78 °C, die Menge der Quenchflüssigkeit im zweiten Kühlkreis 1 000 Teile pro Stunde.

In Form einer 40-gewichtsprozentigen Glyoxallösung werden über die Leitung (27) 60 Teile Lösung oder 24 Teile pro Stunde Glyoxal entnommen, entsprechend einer zweiten Teilausbeute von 10,3 % der Theorie. Die Glyoxallösung hat einen Gehalt von 0,05 Gewichtsprozent Glykol und 14,8 Gewichtsprozent Formaldehyd, 5 Gewichtsprozent Glykolaldehyd, 0,05 Gewichtsprozent Glykolsäure, 3,5 Gewichtsprozent Ameisensäure, 0,05 Gewichtsprozent Glyoxylsäure. Farbzahl der Lösung nach dreitägigem Betrieb : 12 (die Farbzahl wird mittels der Platin-Cobalt-Skala nach ASTM D 1209-69 bestimmt). Auch während einer Betriebszeit von 30 Tagen bilden sich keine festen Rückstände.

## Anspruch

Verfahren zur kontinuierlichen Herstellung von Glyoxal durch Oxidation von Glykol in Gegenwart eines Silberkatalysators bei erhöhter Temperatur, Abkühlung der heißen Reaktionsgase und Abtrennung des Endstoffs, dadurch gekennzeichnet, daß man aus dem 450 bis 800 °C heißen Strom des dampfförmigen Reaktionsgemisches nach der Oxidation und höchstens eine Sekunde nach Austritt aus dem Katalysator mittels Wasser oder wäßriger Glyoxallösung von einer Temperatur von 0 bis 130 °C in Form von Tröpfchen von einem durchschnittlichen Durchmesser von 1 bis 2 000 Mikrometern Glyoxal, Glykol und/oder Glykolaldehyd, zusammen mit Wasser und gewünschtenfalls Formaldehyd, ganz oder teilweise kondensiert, wobei die Hauptmenge der Tröpfchen auf den Strom der Reaktionsgase mit einem Winkel von 2 bis 85° zur Stromachse auftrifft.

## Claim

A process for the continuous preparation of glyoxal by oxidizing glycol in the presence of a silver catalyst at elevated temperature, cooling the hot reaction gases and separating off the end product, wherein all or part of the glyoxal, glycol and/or glycol aldehyde, together with water and, if desired, formaldehyde are condensed out of the reaction mixture, which is in vapor form and at from 450 to 800 °C, after the oxidation and not more than one second after the gases have issued from the catalyst, by means of water or aqueous glyoxal solution at from 0° to 130 °C and in the form of droplets having a mean diameter of from 1 to 2,000 micrometers, the majority of the droplets impinging on the stream of reaction gases at an angle of from 2° to 85° to the axis of the stream.

## Revendication

Procédé pour la préparation en continu du glyoxal par oxydation de glycol à température élevée en présence d'un catalyseur à l'argent, refroidissement des gaz réactionnels chauds et séparation du produit final, caractérisé en ce que, du courant du mélange réactionnel gazeux, se trouvant à une température entre 450 et 800 °C, on extrait par condensation, après l'oxydation et tout au plus 1 seconde après la sortie de la couche de catalyseur, la totalité ou une partie du glyoxal, du glycol et (ou) de l'aldéhyde glycolique, ainsi que de l'eau et éventuellement de l'aldéhyde formique, à l'aide d'eau ou d'une solution aqueuse de glyoxal se trouvant à une température comprise entre 0 et 130 °C, sous forme de gouttelettes avec un diamètre moyen de 1 à 2 000 micromètres, la fraction principale des gouttelettes étant projetée sur le courant des gaz réactionnels sous un angle de 2 à 85° par rapport à l'axe du courant.

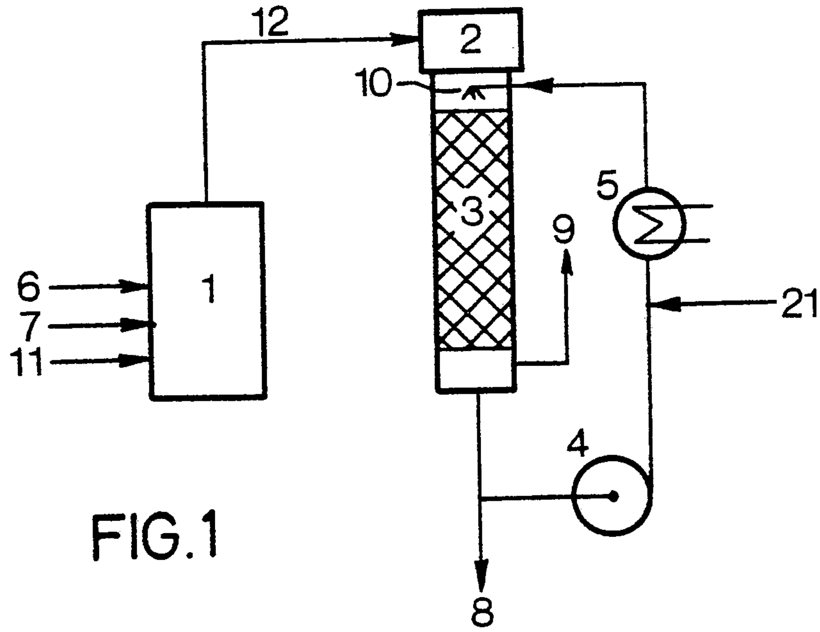


FIG.1

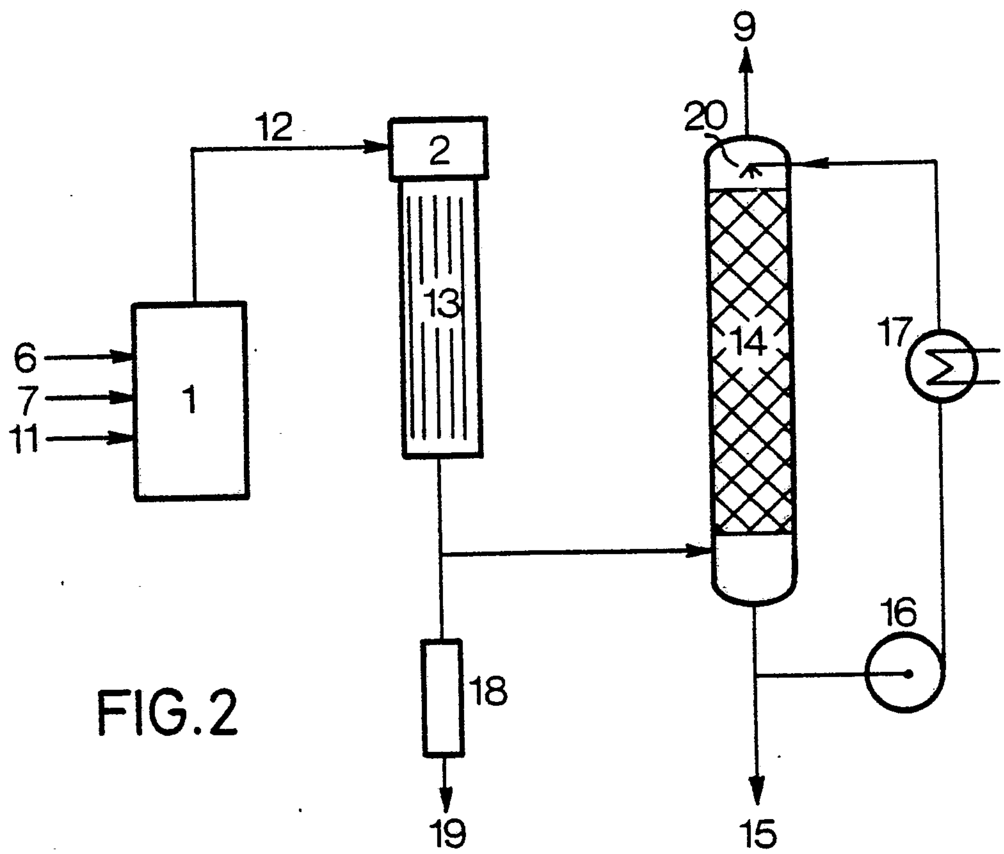


FIG.2

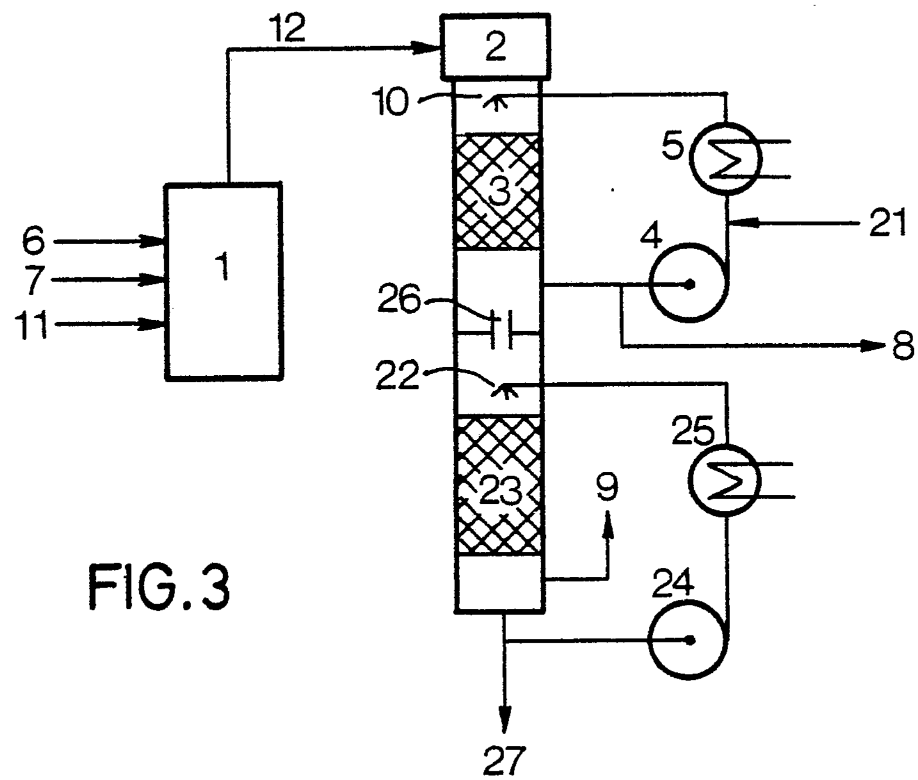


FIG.3